(19)

Europäisches Patentamt

European Patent Office

Office européen des brevets

(11) **EP 3 664 098 A1**

(12) **EUROPEAN PATENT APPLICATION**

(43) Date of publication:
**10.06.2020 Bulletin 2020/24**

(21) Application number: **19175203.9**

(22) Date of filing: **17.05.2019**

(51) Int Cl.:
**G16H 30/40** *(2018.01)* **G16H 50/70** *(2018.01)*
**G16H 50/20** *(2018.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.12.2018 TW 107143784**

(71) Applicant: **Acer Incorporated New Taipei City 221 (TW)**

(72) Inventors:
- **Huang, Chun-Kai**
  **221 New Taipei City (TW)**
- **Li, Ai-Hsien**
  **221 New Taipei City (TW)**
- **Hsiao, Yen-Ju**
  **221 New Taipei City (TW)**
- **Lin, Yun-Ting**
  **221 New Taipei City (TW)**

(74) Representative: **2K Patentanwälte Blasberg Kewitz & Reichel Partnerschaft mbB Schumannstrasse 27 60325 Frankfurt am Main (DE)**

(54) **METHOD AND SYSTEM FOR EVALUATING CARDIAC STATUS, ELECTRONIC DEVICE AND ULTRASONIC SCANNING DEVICE**

(57)     A method and a system for evaluating a cardiac status, an electronic device and an ultrasonic scanning device are provided. The method includes: obtaining at least one first image, wherein each of the at least one first image is a two-dimensional image and includes a first cardiac image; training a depth learning model by the first image; and analyzing at least one second image by using the trained depth learning model to automatically evaluate a cardiac status of a user, wherein each of the at least one second image is the two-dimensional image and includes a second cardiac image.

FIG. 6

EP 3 664 098 A1

## Description

BACKGROUND

Technical Field

The disclosure relates to a physiological status evaluation technology, and particularly relates to a method and a system for evaluating cardiac status, an electronic device and an ultrasonic scanning device.

Description of Related Art

Cardiac ultrasonic image may reflect a structure and a function of a heart, for example, to indicate a size, a contraction status of the heart and/or a heart valve activity. The cardiac ultrasonic image may be a two-dimensional (2D) image or a three-dimensional (3D) image. Information provided by a 2D ultrasonic image is obviously less than information provided by a 3D ultrasonic image. For example, the 2D ultrasonic image cannot provide depth information of the image, and the 3D ultrasonic image has integral depth information, so as to more accurately evaluate a cardiac status. However, equipment for capturing the 3D ultrasonic image is very expensive, which is not popularized in use. Therefore, how to more conveniently provide evaluation information of the cardiac status based on the 2D ultrasonic image is one of subjects studied by those skilled in the art in the technical field.

SUMMARY

The disclosure is directed to a method and a system for evaluating cardiac status, an electronic device and an ultrasonic scanning device, which are adapted to automatically evaluate a cardiac status of a user based on 2D ultrasonic images, so as to effectively ameliorate a usage rate of a 2D ultrasonic scanning device.

An embodiment of the disclosure provides a method for evaluating cardiac status including: obtaining at least one first image, wherein each of the at least one first image is a two-dimensional image and includes a first cardiac pattern; training a depth learning model by using the first image; and analyzing at least one second image by using the trained depth learning model to automatically evaluate a cardiac status of a user, wherein each of the at least one second image is the two-dimensional image and includes a second cardiac pattern.

An embodiment of the disclosure provides an electronic device including a storage device and a processor. The storage device is configured to store at least one first image and at least one second image. Each of the at least one first image is a two-dimensional image and includes a first cardiac pattern, and each of the at least one second image is the two-dimensional image and includes a second cardiac pattern. The processor is coupled to the storage device. The processor trains a depth learning model by using the first image. The processor analyzes the at least one second image by using the trained depth learning model to automatically evaluate a cardiac status of a user.

An embodiment of the disclosure provides a cardiac status evaluation system including an ultrasonic scanning device and an electronic device. The ultrasonic scanning device is configured to execute an ultrasonic scanning to a user to obtain at least one image. Each of the at least one image is a two-dimensional image and includes a cardiac pattern. The electronic device is coupled to the ultrasonic scanning device. The electronic device analyzes the image by using a depth learning model to automatically evaluate a cardiac status of the user.

An embodiment of the disclosure provides an ultrasonic scanning device including an ultrasonic scanner and a processor. The ultrasonic scanner is configured to execute an ultrasonic scanning to a user to obtain at least one image. Each of the at least one image is a two-dimensional image and includes a cardiac pattern. The processor is coupled to the ultrasonic scanner. The processor analyzes the image by using a depth learning model to automatically evaluate a cardiac status of the user.

According to the above description, the 2D ultrasonic image including the cardiac pattern of the user may be analyzed by the depth learning model, so as to automatically evaluate the cardiac status of the user. Moreover, the depth learning model may be trained by the 2D ultrasonic images including the cardiac patterns, so as to improve evaluation accuracy. In this way, a usage rate of 2D ultrasonic scanning devices may be effectively enhanced, so as to reduce setting cost of the ultrasonic scanning device.

To make the aforementioned more comprehensible, several embodiments accompanied with drawings are described in detail as follows.

BRIEF DESCRIPTION OF THE DRAWINGS

The accompanying drawings are included to provide a further understanding of the disclosure, and are incorporated in and constitute a part of this specification. The drawings illustrate embodiments of the disclosure and, together with the description, serve to explain the principles of the disclosure.

FIG. 1 is a schematic diagram of a cardiac status evaluation system according to an embodiment of the disclosure.
FIG. 2 is a schematic diagram of training a depth learning model according to an embodiment of the disclosure.
FIG. 3 is a schematic diagram of analyzing ultrasonic images according to an embodiment of the disclosure.
FIG. 4 and FIG. 5 are schematic diagrams of ultrasonic images according to an embodiment of the disclosure.
FIG. 6 is a flowchart illustrating a method for evaluating cardiac status according to an embodiment of the disclosure.

DESCRIPTION OF THE EMBODIMENTS

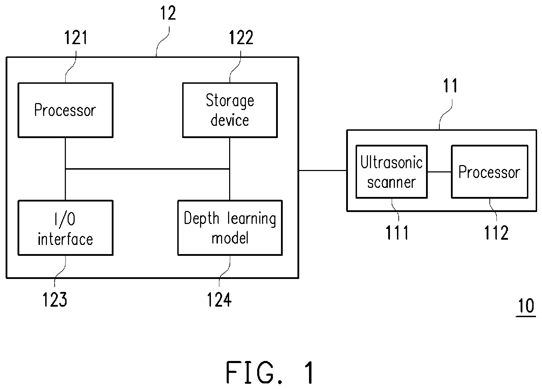

FIG. 1 is a schematic diagram of a cardiac status evaluation system according to an embodiment of the disclosure. Referring to FIG. 1, the system (which is also referred to as the cardiac status evaluation system) 10 includes an ultrasonic scanning device 11 and an electronic device 12. The ultrasonic scanning device 11 may be connected to the electronic device 12 through a wired or wireless manner.

The ultrasonic scanning device 11 is configured to execute an ultrasonic scanning on a body of a user to obtain at least one ultrasonic image reflecting a structure and/or a function of at least one body organ of the user. For example, after the user's heart is scanned by the ultrasonic scanning device 11, the ultrasonic image including a cardiac pattern is obtained. The cardiac pattern may reflect a structure and/or a function of the heart of the user. In an embodiment, the ultrasonic scanning device 11 may also be used for scanning other body parts of the user to obtain the corresponding ultrasonic images, which is not limited by the disclosure.

It should be noted that in the following embodiments, a two-dimensional (2D) ultrasonic scanning device is applied to serve as the ultrasonic scanning device 11. For example, the ultrasonic scanning device 11 may be used for executing 2D ultrasonic scanning to the body of the user to obtain a 2D ultrasonic image. However, in another embodiment, the ultrasonic scanning device 11 may also be a 3D ultrasonic scanning device, which is not limited by the disclosure.

The ultrasonic scanning device 11 may include an ultrasonic scanner 111 and a processor 112. The ultrasonic scanner 111 is configured to execute ultrasonic scanning to the body of the user. The processor 112 is coupled to the ultrasonic scanner 111. The processor 112 may be a Central Processing Unit (CPU), a graphics processor or other programmable general purpose or special purpose microprocessor, a Digital Signal Processor (DSP), a programmable controller, an Application Specific Integrated Circuits (ASIC), a Programmable Logic Device (PLD), or other similar device or a combination of the above devices.

The processor 112 may control an overall or a partial operation of the ultrasonic scanning device 11. In an embodiment, the processor 112 may control the ultrasonic scanner 111 to execute the ultrasonic scanning. In an embodiment, the processor 112 may generate an ultrasonic image according to a scanning result of the ultrasonic scanner 111.

The electronic device 12 may be a notebook computer, a desktop computer, a tablet computer, an industrial computer, a server or a smart phone, etc., that has a data transmission function, a data storage function and a data computation function. The type and the number of the electronic device 12 are not limited by the disclosure. In an embodiment, the electronic device 12 and the ultrasonic scanning device 11 may also be combined into one single device.

The electronic device 12 includes a processor 121, a storage device 122, an input/output interface 123 and a depth learning model 124. The processor 121 may be a CPU, a graphics processor or other programmable general purpose or special purpose microprocessor, a DSP, a programmable controller, an ASIC, a PLD, or other similar device or a combination of the above devices. The processor 121 may control an overall or partial operation of the electronic device 12.

The storage device 122 is coupled to the processor 121. The storage device 122 is used for storing data. For example, the storage device 122 may include a volatile storage medium and a non-volatile storage medium, where the volatile storage medium may be a Random Access Memory (RAM), and the non-volatile storage medium may be a Read Only Memory (ROM), a Solid State Drive (SSD) or a conventional hard drive.

The input/output interface 123 is coupled to the processor 121. The input/output interface 123 is used for receiving signals and/or outputting signals. For example, the input/output interface 123 may include a screen, a touch screen, a touch panel, a mouse, a keyboard, a physical key, a speaker, a microphone, a wired communication interface and/or a wireless communication interface, and the type of the input/output interface 123 is not limited thereto.

The depth learning model 124 may be implemented by software or hardware. In an embodiment, the depth learning model 124 may be implemented by a hardware circuit. For example, the depth learning model 124 may be a CPU, a graphics processor or other programmable general purpose or special purpose microprocessor, a DSP, a programmable controller, an ASIC, a PLD, or other similar device or a combination of the above devices. In an embodiment, the depth learning model 124 may be implemented by a software circuit. For example, the depth learning model 124 may be program codes stored in the storage device 122. The depth learning model 124 may be executed by the processor 121. Moreover, the depth learning model 124 may be a Convolutional Neural Networks (CNN) or other types of neural networks.

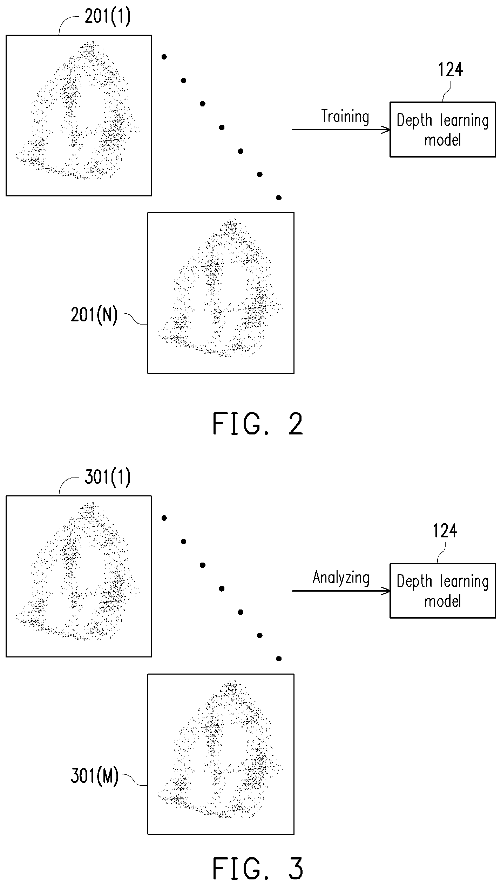

FIG. 2 is a schematic diagram of training the depth learning model according to an embodiment of the disclosure. Referring to FIG. 1 and FIG. 2, the processor 121 may obtain ultrasonic images (which are also referred to as first images) 201(1)-201(N), where N is an arbitrary positive integer. Each of the ultrasonic images 201(1)-201(N) is a 2D image and includes a cardiac pattern (which is also referred to as a first cardiac pattern). For example, at least one of the ultrasonic images 201(1)-201(N) may be obtained by performing ultrasonic scanning on heart portions of one or a plurality of human bodies. The ultrasonic images 201(1)-201(N) may have one single resolution or at least two different resolutions. The first cardiac patterns in the ultrasonic images 201(1)-201(N) may have one or a plurality of sizes. Moreover, the ultrasonic images 201(1)-201(N) may be obtained by performing ultrasonic scanning on heart portions of a human body in different angles.

The processor 121 may train the depth learning model 124 by using the ultrasonic images 201(1)-201(N). For example, regarding the ultrasonic image 201(1), the depth learning model 124 may automatically detect an edge and/or a position of a specific part in the cardiac pattern. For example, the specific part may include a left ventricle, a right ventricle, a left atrium, a right atrium and/or a mitral valve, and the specific part may also include other portions of the heart. The depth learning model 124 may compare a detection result with a correct result to gradually improve image recognition capability. In other words, the trained depth learning model 124 may gradually increase the recognition ability for the cardiac patterns in the ultrasonic images.

FIG. 3 is a schematic diagram of analyzing the ultrasonic images according to an embodiment of the disclosure. Referring to FIG. 1 and FIG. 3, the processor 121 may obtain ultrasonic images (which are also referred to as second images) 301(1)-301(M), where M is an arbitrary positive integer. Each of the ultrasonic images 301(1)-301(M) is a 2D image and includes a cardiac pattern (which is also referred to as a second cardiac pattern). For example, the ultrasonic images 301(1)-301(M) may be obtained by using the ultrasonic scanning device 11 to perform ultrasonic scanning on a heart portion of one single user (which is also referred to as a target user). The ultrasonic images 301(1)-301(M) may have one single resolution or at least two different resolutions. The second cardiac patterns in the ultrasonic images 301(1)-301(M) may have one or a plurality of sizes. Moreover, the ultrasonic images 301(1)-301(M) may be obtained by performing ultrasonic scanning on the heart portion of the target user in different angles.

The trained depth learning model 124 may be used for analyzing the ultrasonic images 301(1)-301(M). For example, the processor 121 may use the depth learning model 124 to analyze the ultrasonic images 301(1)-301(M) to automatically evaluate a cardiac status of the target user. For example, regarding the ultrasonic image 301(1), the depth learning model 124 may automatically detect an edge and/or a position of a specific part in the cardiac pattern. For example, the specific part may include a left ventricle, a right ventricle, a left atrium, a right atrium and/or a mitral valve, and the specific part may also include other portions of the heart. The processor 121 may automatically evaluate the cardiac status of the target user according to the detection result.

The processor 121 may use the depth learning model 124 to analyze the ultrasonic images 301(1)-301(M) and generate an evaluation result. The evaluation result may reflect the cardiac status of the target user. In an embodiment, the evaluation result may reflect at least one of an end-diastolic volume, an end-systolic volume, a left ventricular boundary, a maximum left ventricular boundary, a minimum left ventricular boundary, an average left ventricular boundary, and a cardiac ejection rate of the heart of the target user (which is also referred to as a target heart). In an embodiment, the evaluation result may reflect a possible physiological status of the target user in the future, for example, ventricular hypertrophy, hypertension and/or heart failure, etc. In an embodiment, the evaluation result may reflect a health status and/or possible defects of the target heart.

In an embodiment, the processor 121 may use the depth learning model 124 to analyze the ultrasonic images 301(1)-301(M) to obtain an end-diastolic volume of the target heart and an end-systolic volume of the target heart. Different combinations of the end-diastolic volume and the end-systolic volume may correspond to different cardiac statuses. The processor 121 may evaluate the cardiac status of the target user according to the end-diastolic volume of the target heart and the end-systolic volume of the target heart. For example, the processor 121 may inquire a database according to the obtained end-diastolic volume and the end-systolic volume to evaluate the cardiac status of the target user. Alternatively, the processor 121 may input the obtained end-diastolic volume and the end-systolic volume to a specific algorithm to evaluate the cardiac status of the target user.

In an embodiment, the processor 121 may use the depth learning model 124 to analyze the ultrasonic images 301(1)-301(M) to automatically detect a maximum left ventricular boundary corresponding to the second cardiac patterns and a minimum left ventricular boundary corresponding to the second cardiac patterns. Then, the processor may respectively obtain the end-diastolic volume of the target heart and the end-systolic volume of the target heart according to the maximum left ventricular boundary and the minimum left ventricular boundary.

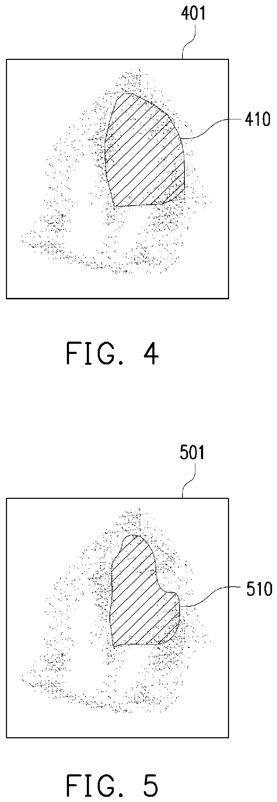

FIG. 4 and FIG. 5 are schematic diagrams of ultrasonic images according to an embodiment of the disclosure. It should be noted that oblique line areas in FIG. 4 and FIG. 5 are left ventricular areas automatically recognized by the depth learning model 124 of FIG. 1. An edge of the oblique line area is a boundary of the left ventricle.

Referring to FIG. 1, FIG. 3, FIG. 4 and FIG. 5, an ultrasonic image 401 is one of the ultrasonic images 301(1)-301(M), and an ultrasonic image 501 is another one of the ultrasonic images 301(1)-301(M). According to the analysis result of the depth learning model 124, the processor 121 may obtain a left ventricular boundary 410 of the ultrasonic image 401 and a left ventricular boundary 510 of the ultrasonic image 501. The left ventricular boundary 410 is the maximum left ventricular boundary (i.e. the maximum left ventricular boundary of the target heart) corresponding to the ultrasonic images 301(1)-301(M). The left ventricular boundary 510 is the minimum left ventricular boundary (i.e. the minimum left ventricular boundary of the target heart) corresponding to the ultrasonic images 301(1)-301(M).

It should be noted that in an embodiment, the depth learning model 124 may automatically recognize a direction of a cardiac pattern in a certain ultrasonic image, for example, a frontal cardiac pattern or a lateral cardiac pattern. The depth learning model 124 may analyze the ultrasonic images 301(1)-301(M) to obtain the maximum left ventricular boundaries of the target heart and the minimum left ventricular boundaries of the target heart in at least two directions. Taking FIG. 4 and FIG. 5 as an example, the left ventricular boundary 410 in the ultrasonic image 401 may be the maximum boundary in a plurality of left ventricular boundaries of the target heart detected in a certain direction (which is also referred to as a first direction), and the left ventricular boundary 510 in the ultrasonic image 501 may be the minimum boundary in the plurality of left ventricular boundaries of the target heart detected in the first direction. The maximum boundary may be used for defining a maximum area of the left ventricle of the target heart. The minimum boundary may be used for defining a minimum area of the left ventricle of the target heart. In other words, in an embodiment, the left ventricular boundary 410 may be a left ventricular boundary when the area of the left ventricle of the target heart is the maximum, and the left ventricular boundary 510 may be a left ventricular boundary when the area of the left ventricle of the target heart is the minimum.

In an embodiment, after the maximum left ventricular boundaries of the target heart and the minimum left ventricular boundaries of the target heart in at least two directions are obtained, the processor 121 may respectively obtain the end-diastolic volume of the target heart and the end-systolic volume of the target heart based on a Simpson's method. For example, the processor 121 may obtain the end-diastolic volume of the target heart or the end-systolic volume of the target heart based on the following equation (1.1).

$$ V = \frac{\pi}{4} \times \sum_{i=1}^{P} a_i b_i \times \frac{L}{P} \qquad \dots (1.1) $$

In the equation (1.1), the parameter V is a volume of the target heart, the parameter $a_i$ is a width (for example, a short axis length) of the left ventricle in the ultrasonic image of the target heart in the first direction (for example, a front view), the parameter $b_i$ is a width (for example, a coronal plane short axis length) of the left ventricle in the ultrasonic image of the target heart in a second direction (for example, a side view), the parameter P may be 20 or other value, and the parameter L is a length (or a long axis length) of the heart. The processor 121 may automatically obtain the required parameters

$a_i$, $b_i$ and L from the ultrasonic images 301(1)-301(M) through the depth learning model 124, so as to calculate the end-diastolic volume of the target heart or the end-systolic volume of the target heart.

In other words, by performing automatic analysis of different angles on the ultrasonic images 301(1)-301(M), even if none of the ultrasonic images 301(1)-301(M) have depth information, the end-diastolic volume of the target heart and the end-systolic volume of the target heart may also be accurately evaluated. Then, the processor 121 may evaluate the cardiac status of the target user according to the end-diastolic volume and the end-systolic volume.

In an embodiment, the processor 121 may obtain a cardiac ejection rate of the target heart according to the end-diastolic volume and the end-systolic volume of the target heart. For example, the processor 121 may obtain the cardiac ejection rate of the target heart according to the following equation (1,2).

$$ EF = \frac{EDV - ESV}{EDV} \times 100\% \qquad \dots (1.2) $$

In the equation (1.2), the parameter EF represents the cardiac ejection rate of the target heart, the parameter EDV represents the end-diastolic volume of the target heart, and the parameter ESV represents the end-systolic volume of the target heart.

In an embodiment, the processor 121 may evaluate the cardiac status of the target user according to the cardiac ejection rate of the target heart, for example, the cardiac ejection rates of different value ranges may correspond to different types of the cardiac status. The processor 121 may evaluate the cardiac status of the target user according to the value range of the cardiac ejection rate of the target heart. For example, the processor 121 may look up a database according to the cardiac ejection rate of the target heart to evaluate the cardiac status of the target user. Alternatively, the processor 121 may input the obtained cardiac ejection rate into a specific algorithm to evaluate the cardiac status of the target user.

It should be noted that in the aforementioned embodiments, the operation of automatically evaluating the cardiac status of the target heart is executed by the processor 121 of the electronic device 12. However, in another embodiment, the operation of automatically evaluating the cardiac status of the target heart may also be executed by the processor 112 of the ultrasonic scanning device 11. For example, the depth learning model 124 may also be implemented in the ultrasonic scanning device 11 and executed by the processor 112. In this way, the ultrasonic scanning device 11 may automatically execute the ultrasonic scanning, the analysis of the ultrasonic images and the evaluation of the cardiac status of the target user. Related operation details have been described above, which are not repeated. Moreover, the depth learning model 124 may be trained by the processor 112 or 121, or trained by other electronic device or server, which is not limited by the disclosure.

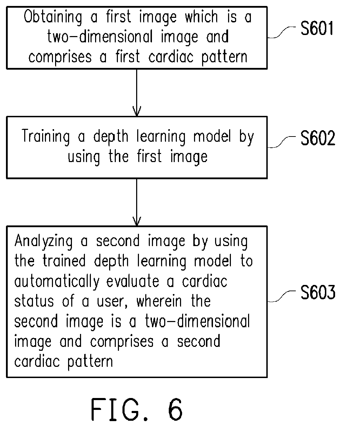

FIG. 6 is a flowchart illustrating a method for evaluating cardiac status according to an embodiment of the disclosure. Referring to FIG. 6, in step S601, at least one first image is obtained. Each of the first images is a 2D image and includes a first cardiac pattern. In step S602, a depth learning model is trained by using the first image. In step S603, at least one second image is analyzed by using the trained depth learning model to automatically evaluate a cardiac status of a user. Each of the second images is the 2D image and includes a second cardiac pattern.

The steps of the method of FIG. 6 have been described above, and details thereof are not repeated. It should be noted that the steps in FIG. 6 may be implemented as a plurality of program codes or circuits, which is not limited by the disclosure. Moreover, the method of FIG. 6 may be used in collaboration with the aforementioned embodiments, or used independently, which is not limited by the disclosure.

In summary, the 2D ultrasonic images including the cardiac patterns of the user may be analyzed by the depth learning model, so as to automatically evaluate the cardiac status of the user. Moreover, the depth learning model may be trained by 2D ultrasonic images including cardiac patterns, so as to improve evaluation accuracy. In this way, a usage efficiency of 2D ultrasonic scanning devices may be effectively enhanced, so as to reduce setting cost of the ultrasonic scanning device. Moreover, the automatically evaluated cardiac status may be used as a reference for medical professionals or non-professionals.

**Claims**

1. A method for evaluating cardiac status, **characterized in that**, the method comprises:

   obtaining (S601) at least one first image (201(1)-201(N)), wherein each of the at least one first image (201(1)-201(N)) is a two-dimensional image and comprises a first cardiac pattern;
   training (S602) a depth learning model (124) by using the at least one first image (201(1)-201(N)); and
   analysing (S603) at least one second image (301(1)-301(M), 401, 501) by using the trained depth learning model (124) to automatically evaluate a cardiac status of a user, wherein each of the at least one second image (301(1)-301(M), 401, 501) is the two-dimensional image and comprises a second cardiac pattern.

2. The method for evaluating cardiac status as claimed in claim 1, wherein the at least one first image (201(1)-201(N)) is obtained through an ultrasonic scanning.

3. The method for evaluating cardiac status as claimed in claim 1, further comprising:
   executing an ultrasonic scanning on the user to obtain the at least one second image (301(1)-301(M), 401, 501).

4. The method for evaluating cardiac status as claimed in claim 1, wherein the step of analysing (S603) the at least one second image (301(1)-301(M), 401, 501) by using the trained depth learning model (124) to automatically evaluate the cardiac status of the user comprises:

   analyzing the at least one second image (301(1)-301(M), 401, 501) to obtain an end-diastolic volume of a heart and an end-systolic volume of the heart; and
   evaluating the cardiac status of the user according to the end-diastolic volume and the end-systolic volume.

5. The method for evaluating cardiac status as claimed in claim 4, wherein the step of analyzing the at least one second image (301(1)-301(M), 401, 501) to obtain the end-diastolic volume of the heart and the end-systolic volume of the heart comprises:

   automatically detecting a maximum left ventricular boundary (410) corresponding to the second cardiac pattern;
   obtaining the end-diastolic volume of the heart according to the maximum left ventricular boundary (410);
   automatically detecting a minimum left ventricular boundary (510) corresponding to the second cardiac pattern; and
   obtaining the end-systolic volume of the heart according to the minimum left ventricular boundary (510).

6. The method for evaluating cardiac status as claimed in claim 4, wherein the step of evaluating the cardiac status of the user according to the end-diastolic volume and the end-systolic volume comprises:

   obtaining a cardiac ejection rate of the heart according to the end-diastolic volume and the end-systolic volume; and
   evaluating the cardiac status of the user according to the cardiac ejection rate.

7. An electronic device (12), **characterized in that**, the electronic device (12) comprises:

   a storage device (122), configured to store at least one first image (201(1)-201(N)) and at least one second image (301(1)-301(M), 401, 501), wherein each of the at least one first image (201(1)-201(N)) is a two-dimensional image and comprises a first cardiac pattern, and each of the at least one second image (301(1)-301(M), 401, 501) is the two-dimensional image and comprises a second cardiac pattern; and a processor (121), coupled to the storage device (122),

wherein the processor (121) trains a depth learning model (124) by using the at least one first image (201(1)-201(N)), and

the processor (121) analyzes the at least one second image (301(1)-301(M), 401, 501) by using the trained depth learning model (124) to automatically evaluate a cardiac status of a user.

8. The electronic device (12) as claimed in claim 7, wherein the at least one first image (201(1)-201(N)) is obtained through an ultrasonic scanning.

9. The electronic device (12) as claimed in claim 7, wherein the processor (121) receives the at least one second image (301(1)-301(M), 401, 501) from an ultrasonic scanning device (11).

10. The electronic device (12) as claimed in claim 7, wherein the operation that the processor (121) analyzes the at least one second image (301(1)-301(M), 401, 501) by using the trained depth learning model (124) to automatically evaluate the cardiac status of the user comprises:

   analyzing the at least one second image (301(1)-301(M), 401, 501) to obtain an end-diastolic volume of a heart and an end-systolic volume of the heart; and
   evaluating the cardiac status of the user according to the end-diastolic volume and the end-systolic volume.

11. The electronic device (12) as claimed in claim 10, wherein the operation that the processor (121) analyzes the at least one second image (301(1)-301(M), 401, 501) to obtain the end-diastolic volume of the heart and the end-systolic volume of the heart comprises:

   automatically detecting a maximum left ventricular boundary (410) corresponding to the second cardiac pattern;
   obtaining the end-diastolic volume of the heart according to the maximum left ventricular boundary (410);
   automatically detecting a minimum left ventricular boundary (510) corresponding to the second cardiac pattern; and

obtaining the end-systolic volume of the heart according to the minimum left ventricular boundary (510).

12. The electronic device (12) as claimed in claim 10, wherein the operation that the processor (121) evaluates the cardiac status of the user according to the end-diastolic volume and the end-systolic volume comprises:

   obtaining a cardiac ejection rate of the heart according to the end-diastolic volume and the end-systolic volume; and
   evaluating the cardiac status of the user according to the cardiac ejection rate.

13. A cardiac status evaluation system (10), **characterized in that**, the cardiac status evaluation system (10) comprises:

   an ultrasonic scanning device (11), configured to execute an ultrasonic scanning to a user to obtain at least one image (301(1)-301(M), 401, 501), wherein each of the at least one image (301(1)-301(M), 401, 501) is a two-dimensional image and comprises a cardiac pattern; and
   an electronic device (12), coupled to the ultrasonic scanning device (11),
   wherein the electronic device (12) analyzes the at least one image (301(1)-301(M), 401, 501) by using a depth learning model (124) to automatically evaluate a cardiac status of the user.

14. The cardiac status evaluation system (10) as claimed in claim 13, wherein the operation that the electronic device (12) analyzes the at least one image (301(1)-301(M), 401, 501) by using the trained depth learning model (124) to automatically evaluate the cardiac status of the user comprises:

   analyzing the at least one image (301(1)-301(M), 401, 501) to obtain an end-diastolic volume of a heart and an end-systolic volume of the heart; and
   evaluating the cardiac status of the user according to the end-diastolic volume and the end-systolic volume.

15. The cardiac status evaluation system (10) as claimed in claim 14, wherein the operation that the electronic device (12) analyzes the at least one image (301(1)-301(M), 401, 501) to obtain the end-diastolic volume of the heart and the end-systolic volume of the heart comprises:

   automatically detecting a maximum left ventricular boundary (410) corresponding to the cardiac pattern;

obtaining the end-diastolic volume of the heart according to the maximum left ventricular boundary (410);
automatically detecting a minimum left ventricular boundary (510) corresponding to the cardiac pattern; and
obtaining the end-systolic volume of the heart according to the minimum left ventricular boundary (510).

16. The cardiac status evaluation system (10) as claimed in claim 14, wherein the operation that the electronic device (12) evaluates the cardiac status of the user according to the end-diastolic volume and the end-systolic volume comprises:

obtaining a cardiac ejection rate of the heart according to the end-diastolic volume and the end-systolic volume; and
evaluating the cardiac status of the user according to the cardiac ejection rate.

17. An ultrasonic scanning device (11), **characterized in that**, the ultrasonic scanning device (11) comprises:

an ultrasonic scanner (111), configured to execute an ultrasonic scanning to a user to obtain at least one image (301(1)-301(M), 401, 501), wherein each of the at least one image (301(1)-301(M), 401, 501) is a two-dimensional image and comprises a cardiac pattern; and
a processor (121), coupled to the ultrasonic scanner (111),
wherein the processor (121) analyzes the at least one image (301(1)-301(M), 401, 501) by using a depth learning model (124) to automatically evaluate a cardiac status of the user.

18. The ultrasonic scanning device (11) as claimed in claim 17, wherein the operation that the processor (121) analyzes the at least one image (301(1)-301(M), 401, 501) by using the trained depth learning model (124) to automatically evaluate the cardiac status of the user comprises:

analyzing the at least one image (301(1)-301(M), 401, 501) to obtain an end-diastolic volume of a heart and an end-systolic volume of the heart; and
evaluating the cardiac status of the user according to the end-diastolic volume and the end-systolic volume.

19. The ultrasonic scanning device (11) as claimed in claim 18, wherein the operation that the processor (121) analyzes the at least one image (301(1)-301(M), 401, 501) to obtain the end-diastolic volume of the heart and the end-systolic volume of the heart comprises:

automatically detecting a maximum left ventricular boundary (410) corresponding to the cardiac pattern;
obtaining the end-diastolic volume of the heart according to the maximum left ventricular boundary (410);
automatically detecting a minimum left ventricular boundary (510) corresponding to the cardiac pattern; and
obtaining the end-systolic volume of the heart according to the minimum left ventricular boundary (510).

20. The ultrasonic scanning device (11) as claimed in claim 18, wherein the operation that the processor (121) evaluates the cardiac status of the user according to the end-diastolic volume and the end-systolic volume comprises:

obtaining a cardiac ejection rate of the heart according to the end-diastolic volume and the end-systolic volume; and
evaluating the cardiac status of the user according to the cardiac ejection rate.

12

121                              122

Processor            Storage
                     device

I/O                  Depth learning
interface            model

123                              124

11

Ultrasonic       Processor
scanner

111              112

10

# FIG. 1

201(1)

201(N)

Training → Depth learning model

124

FIG. 2

301(1)

301(M)

Analyzing → Depth learning model

124

FIG. 3

401

410

FIG. 4

501

510

FIG. 5

Obtaining a first image which is a
two-dimensional image and
comprises a first cardiac pattern — S601

Training a depth learning model by
using the first image — S602

Analyzing a second image by using
the trained depth learning model to
automatically evaluate a cardiac
status of a user, wherein the
second image is a two-dimensional
image and comprises a second
cardiac pattern — S603

FIG. 6

Europäisches
Patentamt

European
Patent Office

Office européen
des brevets

## EUROPEAN SEARCH REPORT

Application Number

EP 19 17 5203

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/360402 A1 (DE JONGE MATTHEW [US] ET AL) 21 December 2017 (2017-12-21) * paragraphs [0106] - [0108], [0171], [0174], [0197], [0274], [0273] - [0288]; figures 5B,13,15A,15B * ----- | 1-20 | INV. G16H30/40 G16H50/70 G16H50/20 |
| X | WO 2017/206023 A1 (SHENZHEN MINDRAY BIO-MEDICAL ELECTRONICS CO LTD [CN]) 7 December 2017 (2017-12-07) * p. 15, l. 23 to p. 16, l. 4; figures 2,3 * ----- | 1,7,13, 17 | |
| X | US 2018/218497 A1 (GOLDEN DANIEL IRVING [US] ET AL) 2 August 2018 (2018-08-02) * paragraphs [0028], [0195], [0333]; figures 6,7,8,46 * ----- | 1,7,13, 17 | |
| X | WO 2017/205836 A1 (ICAHN SCHOOL MED MOUNT SINAI [US]; KHADER SHAMEER [US] ET AL.) 30 November 2017 (2017-11-30) * paragraphs [0012], [0016], [0049], [0050], [0092] * ----- | 1,7,13, 17 | TECHNICAL FIELDS SEARCHED (IPC)  G16H |
| X | US 2017/109881 A1 (AVENDI MICHAEL RASHIDI [US] ET AL) 20 April 2017 (2017-04-20) * paragraphs [0002], [0155], [0158] - [0161] * ----- | 1,7,13, 17 | |

The present search report has been drawn up for all claims

| Place of search | Date of completion of the search | Examiner |
|---|---|---|
| Munich | 27 November 2019 | Heidrich, Alexander |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.                    EP 19 17 5203

This annex lists the patent family members relating to the patent documents cited in the above-mentioned European search report.
The members are as contained in the European Patent Office EDP file on
The European Patent Office is in no way liable for these particulars which are merely given for the purpose of information.

27-11-2019

| Patent document cited in search report | Publication date | Patent family member(s) | Publication date |
|---|---|---|---|
| US 2017360402 A1 | 21-12-2017 | AU 2017281281 A1 | 06-12-2018 |
| | | CA 3026162 A1 | 28-12-2017 |
| | | CN 109310396 A | 05-02-2019 |
| | | EP 3471623 A1 | 24-04-2019 |
| | | JP 2019521745 A | 08-08-2019 |
| | | KR 20190021344 A | 05-03-2019 |
| | | TW 201800057 A | 01-01-2018 |
| | | US 2017360401 A1 | 21-12-2017 |
| | | US 2017360402 A1 | 21-12-2017 |
| | | US 2017360403 A1 | 21-12-2017 |
| | | US 2017360404 A1 | 21-12-2017 |
| | | US 2017360411 A1 | 21-12-2017 |
| | | US 2017360412 A1 | 21-12-2017 |
| | | WO 2017222970 A1 | 28-12-2017 |
| WO 2017206023 A1 | 07-12-2017 | CN 108882917 A | 23-11-2018 |
| | | WO 2017206023 A1 | 07-12-2017 |
| US 2018218497 A1 | 02-08-2018 | CN 110475505 A | 19-11-2019 |
| | | EP 3573520 A2 | 04-12-2019 |
| | | US 2018218497 A1 | 02-08-2018 |
| | | US 2018218502 A1 | 02-08-2018 |
| | | WO 2018140596 A2 | 02-08-2018 |
| WO 2017205836 A1 | 30-11-2017 | NONE | |
| US 2017109881 A1 | 20-04-2017 | NONE | |

EPO FORM P0459

For more details about this annex : see Official Journal of the European Patent Office, No. 12/82